# EUROPEAN PATENT SPECIFICATION

(11) **EP 2 892 349 B1**
(45) Date of publication and mention of the grant of the patent: **23.08.2017**
(21) Application number: 13744999.7
(22) Date of filing: 22.07.2013
(51) Int. Cl.: A01N 59/16, B82Y 30/00, C09D 7/12, C08F 8/42, C23C 14/08, C23C 14/20, A01N 25/10

(54) **PHOTOCATALYTIC TiO2 COATINGS ON THE POLYMER SURFACES ACTIVATED WITH VISIBLE LIGHT, METHOD OF THEIR PREPARATION AND USE THEREOF**
DURCH SICHTBARES LICHT AKTIVIERTE, PHOTOKATALYTISCHE TIO2-BESCHICHTUNGEN AUF POLYMEROBERFLÄCHEN, VERFAHREN ZU DEREN HERSTELLUNG UND VERWENDUNG DAVON
REVÊTEMENTS PHOTOCATALYTIQUES EN TiO2 SUR LES SURFACES POLYMÈRES ACTIVÉES PAR LA LUMIÈRE VISIBLE, LEUR PROCÉDÉ DE PRÉPARATION ET LEUR UTILISATION

(30) Priority: 23.07.2012 PL 40009812
(43) Date of publication of application: 15.07.2015
(73) Proprietor: Uniwersytet Jagiellonski, 31007 Krakow (PL)
(72) Inventor: MACYK, Wojciech, PL-32-060 Liszki (PL); SADOWSKI, Rafal, PL-30-322 Kraków (PL); LABUZ, Przemyslaw, PL-31-626 Kraków (PL); BUCHALSKA, Marta, PL-30-392 Kraków (PL)
(74) Representative: Witek, Rafal
(86) International application number: PCT/EP2013/065400
(87) International publication number: WO 2014/016239

(56) References cited:
- WO-A2-2010/098687
- US-A1- 2009 068 449
- DATABASE WPI Week 200525 Thomson Scientific, London, GB; AN 2005-237134 XP002715831, & JP 2005 068185 A (KAWASAKI HEAVY IND LTD) 17 March 2005 (2005-03-17)
- J. KASANEN ET AL.: "Self-cleaning, titanium dioxide based, multilayer coating fabricated on polymer and glass surfaces", J. APPL. POLYM. SCI., vol. 111, 2009, pages 2597-2606, XP8152425, cited in the application

## Description

### Technical field

The subject of the invention are photocatalytic titanium oxide(IV) coatings on the polymer surfaces activated with visible light, method for their preparation and their use. The polymer surfaces are selected from the group of polymers used in medical and food packaging, in particular polyurethane (PU), polycarbonate (PC), polyvinyl chloride (PVC), polytetrafluoroethylene (PTFE).

### State of art

There has been a significant increase in the use of plastics in almost every field of human activity in recent years. The low price and simplicity of adaptation of materials to the requirements of a developing society is contributed to this particularly. Polymeric materials also play an increasingly important role in medicine, since the disposable medical equipment is made of these materials. The surface of the plastics is exposed to the formation of biofilm what is dangerous to health and what is one of the most important etiological contribution to the nosocomial infections. According to World Health Organization, the minimization of the nosocomial infections positively impacts on the patients' health as well as on the economic condition of medical facilities. The answer to the problem of nosocomial infections is to design new materials with potential antibacterial effect. The increase in resistance of pathogens to conventionally available antimicrobial agents requires exploration of other innovative solutions.

The answer to the problem is utilization of photocatalytic materials, particularly semiconductors, for example titanium oxide(IV), in photoinactivation of the bacteria. The present state of art discloses such applications, however, titanium oxide(IV) usually occurs in the form of powders, solutions or colloidal layers at mineral surfaces such as glass or metal (Chen, X., Mao, S.S, 2007, Chemical Reviews, 107, 2891-2959), what significantly reduces the possibility of its application.

The activation process of the polymer surfaces by low-temperature plasma technique is well known and described in the literature by Hegemann (D. Hegemann et al., Nuclear Instruments and Methods in Physics Research B 2003, 208, 281-286), Kasanen (J. Kasanen et al., 2009, J. Appl. Polym. Sci., 111, 2597-2606) and Vandencasteele (N. Vandencasteele et al., 2010, Journal of Electron Spectroscopy and Related Phenomena, 178-179, 394-408).The activation of the polymer surfaces changes their physical and chemical properties. As a result of the activation process, many oxygen-containing functional groups are generated at the surface of polymeric material in the presence of oxygen, such as hydroxyl group (-OH), carboxyl group (-COOH), carbonyl group (-C=O).

The methods of modifying of the material properties without the changes in volume by applying plasma treatment are also known. For instance the plasma treatment used for modifying the optical fiber cable is disclosed in patent application GB 248892 A. The entire length of optical fiber is subjected to plasma treatment by moving it through the plasma furnace, thereupon the surface of plasma-treated optical fiber is modified becoming more susceptible to combining it with the target substance. The method allows for creation a layer of target substance adhering to the surface of optical fiber cable. The materials used in abovementioned example for the adhering layer formation are epoxy resins, polyamide and polyurethane.

WO 2010/098687 A2 discloses colloidal solutions of nanocrystalline TiO₂ which is surface modified with organic compounds which is photocatalytically active in visible light.

JP 2005 068 185 A discloses fluorine type polymers which are plasma treated and subsequently coated with a TiO₂ slurry. Inconvenience observed in the prior art relates to the difficulty in obtaining sustained coatings of titanium oxide(IV) on the polymer surfaces and their sensitization to the visible light range. Permanent immobilization of titanium oxide(IV) on the polymer surfaces provides new opportunities for the application of photocatalytic processes at TiO₂, for example to photosterilize of various medical materials made of plastics. The aim of the present invention is to provide sustained photocatalytic coatings of titanium oxide(IV) on the polymer surfaces, activated with visible or ultraviolet light, exhibiting high photocatalytic and photosterilizing activity and the method of their preparation. The present invention provides also new opportunities for the application of coatings according to the invention, a certain set of characteristics to the sterilization of various materials, such as medical catheters or food packaging.

### Detailed description of the invention

The invention relates to the photocatalytic coatings of titanium oxide(IV) on the polymer surfaces activated with visible light, characterized by:
a) their application on organic substrate which is a polymeric material used in medicine, food and pharmaceuticals packaging, in particular selected from the group of: polyurethane (PU), polycarbonate (PC), polyvinyl chloride (PVC), polytetrafluoroethylene (PTFE).
b) coating of nanocrystalline titanium oxide(IV) or nanocrystalline titanium oxide(IV) surface-modified with organic compounds selected from the group consisting of: phthalic acid, 4-sulfophthalic acid, 4-amino-2-hydroxybenzoic acid, 3-hydroxy-2-naphthyl acid, salicylic acid, 6-hydroxysalicylic acid, 5-hydroxysalicylic acid, 5-sulfosalicylic acid, 3,5-dinitrosalicylic acid, 1,4-dihydroxy-1,3-benzenedisulfonic disodium salt, gallic acid, pyrogallol, 2,3-naphthalenediol, 4-methylcatechol, 3,5-di-tert-butylcatechol, p-nitrocatechol, 3,4-dihydroxy-L-phenylalanine (DOPA), catechol, rutoside and ascorbic acid.

The invention relates also to the method of preparation of titanium oxide(IV) photocatalytic coatings activated with visible light on the surfaces of the polymers used in medicine, food and pharmaceuticals packaging, in particular selected from the group of: polyurethane (PU), polycarbonate (PC), polyvinyl chloride (PVC), polytetrafluoroethylene (PTFE) and is characterized in that it comprises three stages:
a) surface activation of a polymer material with low temperature plasma technique,
b) synthesis of nanocrystalline coating of titanium oxide(IV) using a suspension of nanocrystalline titanium oxide(IV),
c) modification of the coating surface using organic compounds by means of application of the modifier in solution.

Preferably, the activation takes place under the influence of the oxygen or nitrogen plasma. Preferably, the activation takes place at a plasma pressure of 0.1-1 mbar with the time of plasma operation between 5-500 s. Also preferably, when polymeric materials activated with plasma are left in the air to form oxygen-containing functional groups.

Also preferably, if nanocrystalline titanium oxide(IV) with a grain size up to 100 nm in the form of an aqueous colloidal solution is used for the synthesis. Preferably, the synthesis process is carried out by the immersion techniques (for instance dip-coating technique), spraying or painting techniques. Preferably, the process takes place at room temperature.

Preferably, surface modification of coating with organic compounds takes place in aqueous or alcoholic solution of modifier with minimum concentration 10⁻⁴ mol/dm³ followed by drying. The organic compound is selected from the group consisting of: phthalic acid, 4-sulfophthalic acid, 4-amino-2-hydroxybenzoic acid, 3-hydroxy-2-naphthyl acid, salicylic acid, 6-hydroxysalicylic acid, 5-hydroxysalicylic acid, 5-sulfosalicylic acid, 3,5-dinitrosalicylic acid (Table 1), 1,4-dihydroxy-1,3-benzenedisulfonic disodium salt, gallic acid, pyrogallol, 2,3-naphthalenediol, 4-methylcatechol, 3,5-di-tert-butylcatechol, p-nitrocatechol, 3,4-dihydroxy-L-phenylalanine (DOPA), catechol (Table 2), rutoside and ascorbic acid.

**Table 1. Phthalic acid and salicylic acid derivatives.**

| Compound symbol | Compound name | Structural formula |
|---|---|---|
| A-1 | phthalic acid | 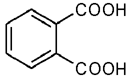 |
| A-2 | 4-sulfophthalic acid | 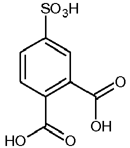 |
| S-1 | 4-am i no-2-hyd roxybenzoic acid | 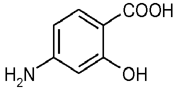 |
| S-2 | 3-hydroxy-2-naphthyl acid | 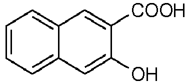 |
| S-3 | salicylic acid | 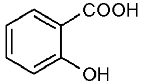 |
| S-4 | 6-hydroxysalicylic acid | 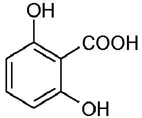 |
| S-5 | 5-hydroxysalicylic acid | 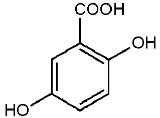 |
| S-6 | 5-sulfosalicylic acid | 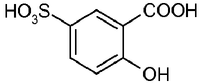 |
| S-7 | 3,5-dinitrosalicylic acid | 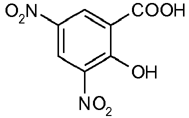 |

**Table 2. Catechol derivatives.**

| Compound symbol | Compound name | Structural formula |
|---|---|---|
| K-1 | 1,4-dihydroxy-1,3-benzenedisulfonic disodium salt | 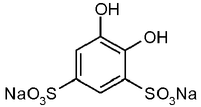 |
| K-2 | gallic acid | 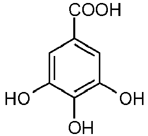 |
| K-3 | pyrogallol | 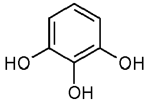 |
| K-4 | 2,3-naphthalenediol | 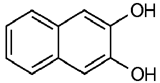 |
| K-5 | 4-methylcatechol | 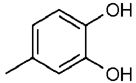 |
| K-6 | 3,5-di-tert-butylcatechol | 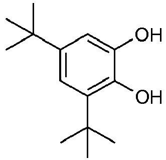 |
| K-7 | p-nitrocatechol | 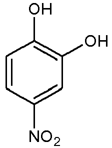 |
| K-8 | 3,4-dihydroxy-L-phenylalanine (DOPA) | 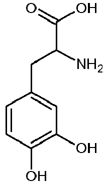 |
| K-9 | 1,2-dihydroxy-benzene (catechol) | 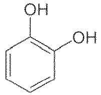 |

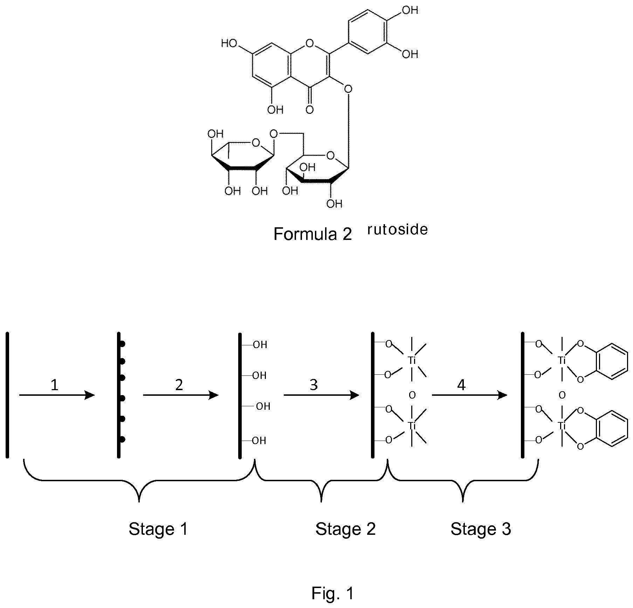

Particular steps of the method are also graphically illustrated in Fig. 1.

Another subject of the invention is the use of the photocatalytic coatings of titanium oxide(IV), as defined above activated with visible light on the surfaces of polymers, and/or obtainable in the method according to the invention, for sterilization of plastic components used in medicine, food and pharmaceuticals packaging, in particular selected from the group of: polyurethane (PU), polycarbonate (PC), polyvinyl chloride (PVC), polytetrafluoroethylene (PTFE).

Particular subject of the invention is use of the coatings according to the invention and/or obtained by the process according to the invention for the production of selected products from the group consisting of: photosterilization materials, photobactericidal, photofungicidal, photocatalytic and other materials, especially transparent, for instance medical catheters, medical plastic tubes, foil and packaging for food and pharmaceuticals.

Particular subject of the invention is use of the coatings according to the invention and/or obtained by the process according to the invention, in medicine (for example in dermatology, ophthalmology, otolaryngology, urology, gynecology, rheumatology, oncology, surgery, hospitality, veterinary medicine and dentistry) and in cosmetology, preferably, for the sterilization of medical catheters, plastic tubes and other surfaces, sterilization which is advantageous and/or required.

### Summary of the invention

The material according to the present invention exhibits photocatalytic activity when irradiated with visible light (*l* > 400 nm; photocatalysis is a consequence of the absorption of visible light by surface complex of titanium, type *charge-transfer*) and ultraviolet light (*l* < 400 nm; photocatalysis is a consequence of the absorption of ultraviolet light by surface complex of titanium, type *charge-transfer* or directly by titanium oxide(IV)). As a result of light exposure, Reactive Oxygen Species are formed (OH^{•}, O₂⁻, H₂O₂, ¹O₂), which are responsible for oxidation of organic compounds and decay of microorganisms.

The present invention in embodiments not limiting the scope of its application is presented in figures 1-6, in which:
- Fig. 1: shows a general diagram of a method for preparing the photocatalytic coating of titanium oxide(IV) activated with visible light, on polymer surfaces,
- Fig. 2: shows UV-vis diffuse-reflectance spectrum of PTFE coated with TiO₂ and modified with various modifiers,

- Fig. 3: shows the transformation of UV-vis diffuse-reflectance spectrum into UV-vis absorbance spectrum of PTFE coated with TiO₂ and modified with various modifiers,
- Fig. 4: shows photodegradation of azure B "dry mode", diffuse-reflectance spectra of TiO₂@PTFE with adsorbed model pollution (azure B), irradiation: XBO-150, water filter, l >320 nm,
- Fig. 5: shows photodegradation of azure B "dry mode", diffuse reflectance spectra of TiO₂@PTFE with adsorbed model pollution (azure B) transformed by Kubleka-Munk function, irradiation: XBO-150, water filter, l >320 nm,
- Fig. 6: shows the surface modification with catechol and salicylic acid, which results in well-defined extension of the scope of activity of the layers to visible light K-9@TiO₂ (b), S-2@TiO₂ (c), K-4@TiO₂ (d) in comparison to the layer made of unmodified TiO₂ (a).

### Example 1.

### Obtaining visible-light active, nanocrystalline photocatalysts as coatings on polymer surfaces.

Starting substrates for the synthesis of described materials are:
a) commercially available plastic materials, particularly polyurethane (PU), polycarbonate (PC), polyvinyl chloride (PVC), polytetrafluoroethylene (PTFE),
b) unmodified, nanocrystalline titanium oxide(IV) in the form of a colloidal, aqueous solution having a particle size less than 100 nm and organic surface modifiers.

### Variant 1

A square with a side length of 3 cm was cut from a commercially available film of polytetrafluoroethylene (PTFE) and purified with detergent. The surface was activated with oxygen plasma in conditions: p = 0.3 mbar, t = 120 s, v = 2 cm³/min, power 100 W. After activation process, the polymer material was left in the air for 2 minutes. The synthesis of the coating was carried out using immersion technique *(dip-coating)* from colloidal solution of nanocrystalline titanium oxide(IV) (15% wt). The drawing speed of polymer material from the solution was established at 1 cm/min at room temperature. As a result, uniform, durable coating of TiO₂ was created at the surface of foil. The resulting coatings were modified using impregnation by immersing the samples for 2 min in a solution of the modifier from the group S (S-2, S-3; Table 1) or group K (K-1, K-4, K-9; Table 2) or ascorbic acid or rutoside in the form of aqueous solution with concentration 10⁻⁴ mol/dm³ and then dried in the air.

### Variant 2

A square with a side length of 3 cm was cut from a commercially available film of polyurethane (PU) and purified with detergent. The surface was activated with oxygen plasma in conditions: p = 0.4 mbar, t = 60 s, v = 2 cm³/min, power 100 W. After activation process, the polymer material was left in the air for 2 minutes. The synthesis of the coating was carried out using immersion technique *(dip-coating)* from colloidal solution of nanocrystalline titanium oxide(IV) (4% wt). The drawing speed of polymer material from the solution was established at 1 cm/min at room temperature. As a result, uniform, durable coating of TiO₂ was created at the surface of foil. The resulting coatings were modified using impregnation by immersing the samples for 2 min in a solution of the modifier from the group S (S-2, S-3; Table 1) or group K (K-1, K-4, K-9; Table 2) or ascorbic acid or rutoside in the form of aqueous solution with concentration 10⁻⁴ mol/dm³ and then dried in the air.

### Variant 3

A square with a side length of 3 cm was cut from a commercially available film of polycarbonate (PC) and purified with detergent. The surface was activated with oxygen plasma in conditions: p = 0.4 mbar, t = 120 s, v = 2 cm³/min, power 100 W. After activation process, the polymer material was left in the air for 2 minutes. The synthesis of the coating was carried out using immersion technique *(dip-coating)* from colloidal solution of nanocrystalline titanium oxide(IV) (15% wt). The drawing speed of polymer material from the solution was established at 1 cm/min at room temperature. As a result, uniform, durable coating of TiO₂ was created at the surface of foil. Prepared coatings were modified using impregnation by immersing the samples for 2 min in a solution of the modifier from the group S (S-2, S-3; Table 1) or group K (K-1, K-4, K-9; Table 2) or ascorbic acid or rutoside in the form of aqueous solution with concentration 10⁻⁴ mol/dm³ and then dried in the air.

### Example 2

### Characterization of obtained materials.

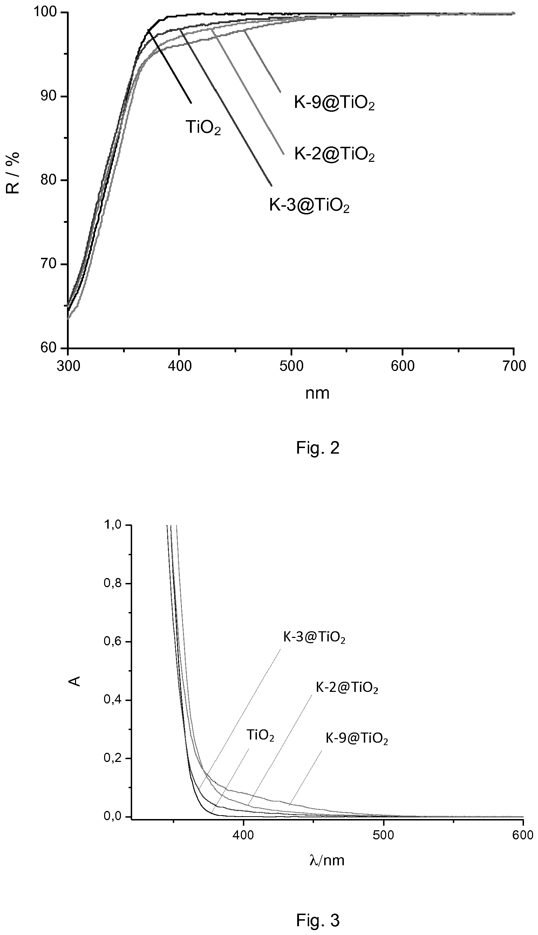

Coatings synthesized according to the Example 1 are permanently affixed to the substrate and indelible. UV-vis spectra of the coatings modified with compounds from the group K are presented in Fig. 2 and Fig. 3. There is a distinct absorption of visible light, preferably in the wavelength range around 500 nm.

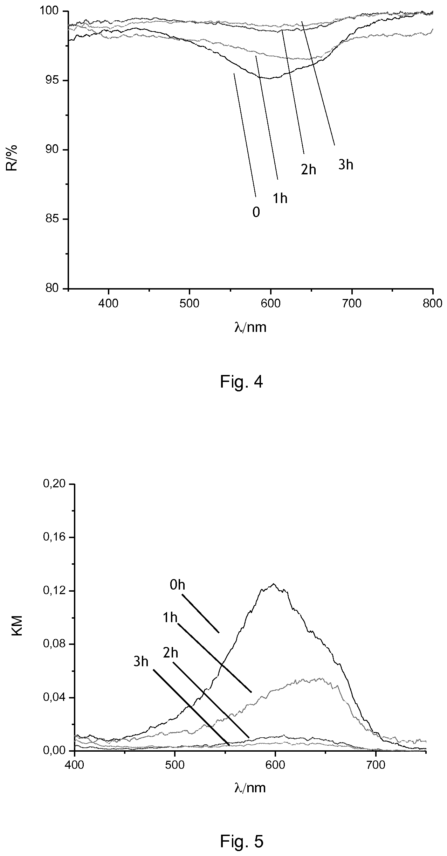

The activity of obtained coatings was specified using "dry mode". Aqueous solutions of azure B were used as model pollution for photocatalytic activity tests. A sample of plastic with the synthesized TiO₂ coating was impregnated in a solution of pigment with a concentration 2.5·10⁻⁴ mol/dm³ for 1 h. Afterwards, the sample was irradiated for 3 h and the changes occurring during the experiment were monitored. The measuring system consisted of the xenon lamp XBO-150. Directly behind the lamp a water filter with copper sulfate(II) solution was placed (which cuts off radiation from the near infrared range, I > 700 nm) and a high-pass filter which transmits radiation in the range I > 320 nm. The sample was placed at a distance of 40 cm from the light source. Changes in the intensity of the dye colour were assessed during irradiation by spectrophotometry. The diffuse-reflectance spectrum and absorbance spectrum of azure B degradation during irradiation are shown in Fig. 4 and Fig. 5. Almost complete degradation of the dye at the time of 3h was observed.

### Example 3

### Studies on photoactivity of obtained coatings.

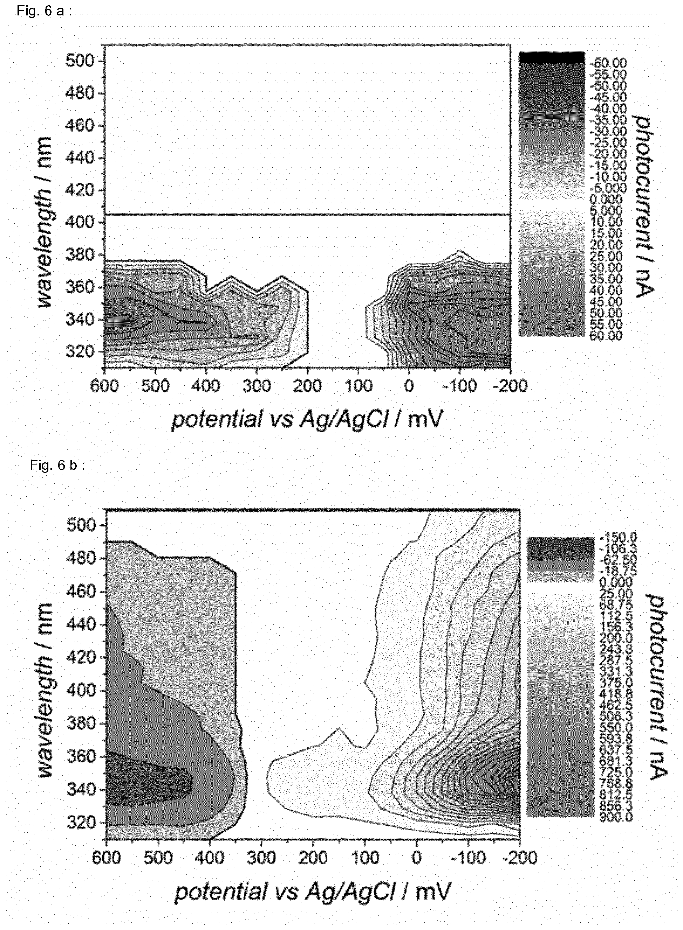

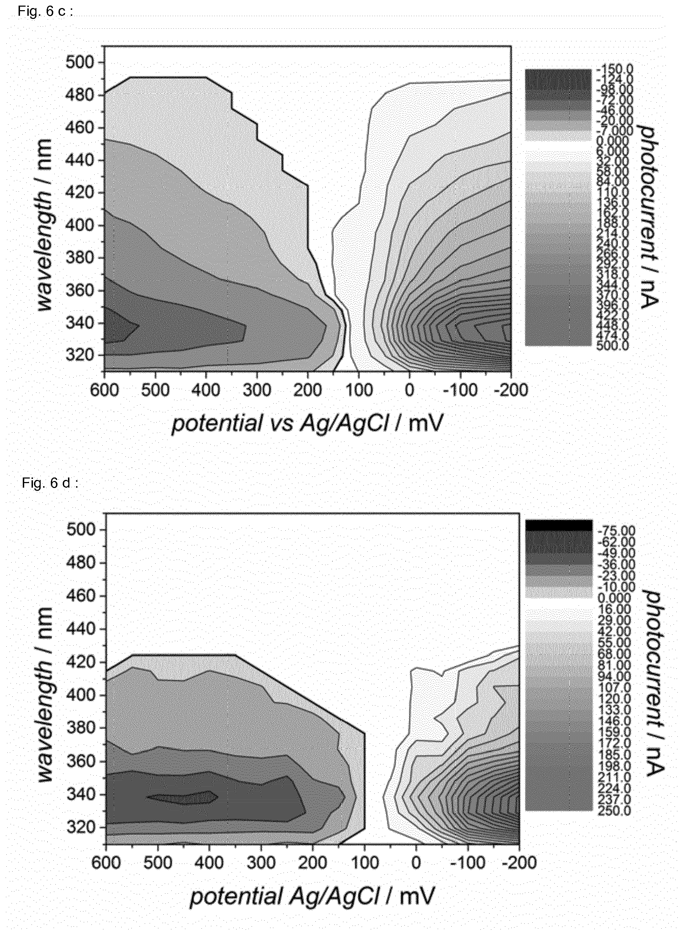

Coatings synthesized according to the Example 1 were tested to determine the spectral range of their activity. For photoelectrochemical measurements the set which included: xenon lamp XBO-150 (Osram) with power 150 W and power supply adaptor LPS-250 (Photon Technology International), monochromator with the shutter and electrochemical analyzer BAS-50W (Bioanalytical systems), was used. The control system of shutter and monochromator allows automatic control of wavelength of the incident light and the time of exposure to radiation of the working electrode. Photoelectrochemical measurements were carried out in three-electrode cell, in quartz cuvette with volume 25 cm³ filled with electrolyte (KNO₃ solution with concentration 0.1 mol dm⁻³). A platinum electrode was used as an auxiliary electrode and silver chloride electrode (Ag/AgCl) - as a reference electrode. Working electrode was the ITO film (transparent electrically conductive material, a mixture of indium-tin oxide) coated with a layer of nanocrystalline TiO₂ modified with organic compounds according to the method described in Example 1. The idea of the photoelectrochemical experiment is to measure the intensity of generated photocurrent as a function of light wavelength (l = f(l)) incident at photoelectrode in the form of short flashes (10 s.), at constant potential applied to the working electrode. The coatings were irradiated with monochromatic light in the wavelength range of 330-700 nm, changing with each flash of fixed interval (9.5 nm). The change in wavelength occurred when the shutter was closed. The plots l = f(l) (share spectra without taking into account the absolute intensity of light) recorded in the potential range 600÷-200 mV were used to determine the photoreply profile as a function of wavelength and potential applied to the working electrode (so called photocurrent maps, I = f(E,I)). Surface modification with catechol derivatives and salicylic acid results in well-defined extension of the scope of activity to visible light (Fig. 6: K-9@TiO₂ (b), S-2@TiO₂ (c), K-4@TiO₂ (d)) in comparison to the coating made of unmodified TiO₂ (Fig. 6a).

## Claims

1. Photocatalytic coating of titanium oxide(IV) on a polymer surface,
**characterized in that:**
a) it is applied to a substrate which is the organic polymer material used in medicine, food and pharmaceutical packing and selected from the group of polyurethane (PU), polycarbonate (PC), polyvinyl chloride (PVC), polytetrafluoroethylene (PTFE),
b) the coating consists of nanocrystalline titanium oxide(IV) surface-modified with organic compounds,
wherein nanocrystalline titanium oxide(IV) is surface-modified with an organic compound from the group consisting of: phthalic acid, 4-sulfophthalic acid, 4-amino-2-hydroxybenzoic acid, 3-hydroxy-2-naphthyl acid, salicylic acid, 6-hydroxysalicylic acid, 5-hydroxysalicylic acid, 5-sulfosalicylic acid, 3,5-dinitrosalicylic acid, 1,4-dihydroxy-1,3-benzenedisulfonic disodium salt, gallic acid, pyrogallol, 2,3-naphthalenediol, 4-methylcatechol, 3,5-di-tert-butylcatechol, p-nitrocatechol, 3,4-dihydroxy-L-phenylalanine (DOPA), catechol, rutoside and ascorbic acid.

2. The method of preparation of titanium oxide(IV) photocatalytic coating on the surface of polymer material used in medicine, food and pharmaceutical packing and selected from the group of: polyurethane (PU), polycarbonate (PC), polyvinyl chloride (PVC), polytetrafluoroethylene (PTFE), **characterized in that** it comprises three stages:
a) surface activation of a polymer material with low temperature plasma technology,
b) synthesis of nanocrystalline coating of titanium oxide(IV) using a suspension of nanocrystalline titanium oxide(IV),
c) modification of the coating surface using organic compounds by means of application of the modifier in solution,
wherein the organic compound used for surface modification is selected from the group consisting of: phthalic acid, 4-sulfophthalic acid, 4-amino-2-hydroxybenzoic acid, 3-hydroxy-2-naphthyl acid, salicylic acid, 6-hydroxysalicylic acid, 5-hydroxysalicylic acid, 5-sulfosalicylic acid, 3,5-dinitrosalicylic acid, 1,4-dihydroxy-1,3-benzenedisulfonic disodium salt, gallic acid, pyrogallol, 2,3-naphthalenediol, 4-methylcatechol, 3,5-di-tert-butylcatechol, p-nitrocatechol, 3,4-dihydroxy-L-phenylalanine (DOPA), catechol, rutoside and ascorbic acid.

3. The method according to claim 2, **characterized in that** activation takes place under the influence of the oxygen or nitrogen plasma.

4. The method according to claims 2 and 3, **characterized in that** activation takes place under plasma pressure of 0,1-1 mbar and in time of plasma operation of 5-500 s.

5. The method according to claim 2, **characterized in that** plasma polymeric materials are left in the air to form oxygen-containing functional groups.

6. The method according to claim 2, **characterized in that** nanocrystalline titanium oxide(IV) with a grain size up to 100 nm in the form of an aqueous colloidal solution is used for the synthesis.

7. The method according to claim 2, **characterized in that** the synthesis process is carried out by the immersion techniques, in particular dip-coating technique, spraying or painting techniques.

8. The method according to claim 2, **characterized in that** the process takes place at room temperature.

9. The method according to claim 2, **characterized in that the** surface modification of coating with organic compounds takes place in aqueous or alcoholic solution of modifier with minimum concentration 10⁻⁴ mol/dm³ followed by drying.

## Patentansprüche

1. Photokatalytische Beschichtung aus Titan(IV)-Oxid auf einer Polymeroberfläche, **dadurch gekennzeichnet, dass**
a) es auf einem Substrat aufgetragen ist, welches das organische Polymermaterial ist, welches für Arzneimittel-, Nahrungsmittel- und pharmazeutische Verpackungen verwendet wird und aus der Gruppe aus Polyurethan (PU), Polycarbonat (PC), Polyvinylchlorid (PVC), Polytetrafluorethylen (PTFE) ausgewählt ist,
b) die Beschichtung aus nanokristallinem Titan(IV)-Oxid, das mit organischen Verbindungen Oberflächen-modifiziert ist, besteht,
wobei nanokristallines Titan(IV)-Oxid mit einer organischen Verbindung aus der Gruppe bestehend aus: Phthalsäure, 4-Sulfophthalsäure, 4-Amino-2-hydroxybenzoesäure, 3-Hydroxy-2-naphthylsäure, Salicylsäure, 6-Hydroxysalicylsäure, 5-Hydroxysalicylsäure, 5-Sulfosalicylsäure, 3,5-Dinitrosalicylsäure, 1,4-Dihydroxy-1,3-benzoldisulfonsäure-dinatriumsalz, Gallussäure, Pyrogallol, 2,3-Naphthalendiol, 4-Methylkatechol, 3,5-Di-tert-butylkatechol, p-Nitrokatechol, 3,4-Dihydroxy-L-phenylalanin (DOPA), Katechol, Rutosid und Ascorbinsäure, Oberflächen-modifiziert ist.

2. Das Verfahren zur Herstellung einer photokatalytischen Titan(IV)-Oxid-Beschichtung auf der Oberfläche eines Polymermaterials, welches für Arzneimittel-, Nahrungsmittel- und pharmazeutische Verpackungen verwendet wird und aus der Gruppe aus: Polyurethan (PU), Polycarbonat (PC), Polyvinylchlorid (PVC), Polytetrafluorethylen (PTFE) ausgewählt ist, **dadurch gekennzeichnet, dass** es drei Stufen umfasst:
a) Oberflächenaktivierung eines Polymermaterials mit Niedertemperatur-plasmatechnologie,
b) Synthese einer nanokristallinen Beschichtung aus Titan(IV)-Oxid unter Verwendung einer Suspension aus nanokristallinem Titan(IV)-Oxid,
c) Modifizierung der Beschichtungsoberfläche unter Verwendung organischer Verbindungen mittels Anwendung des Modifikators in Lösung,
wobei die organische Verbindung, die zur Oberflächenmodifizierung verwendet wird, aus der Gruppe bestehend aus: Phthalsäure, 4-Sulfophthalsäure, 4-Amino-2-hydroxybenzoesäure, 3-Hydroxy-2-naphthylsäure, Salicylsäure, 6-Hydroxysalicylsäure, 5-Hydroxysalicylsäure, 5-Sulfosalicylsäure, 3,5-Dinitrosalicylsäure, 1,4-Dihydroxy-1,3-benzoldisulfonsäure-dinatriumsalz, Gallussäure, Pyrogallol, 2,3-Naphthalendiol, 4-Methylkatechol, 3,5-Di-tert-butylkatechol, p-Nitrokatechol, 3,4-Dihydroxy-L-phenylalanin (DOPA), Katechol, Rutosid und Ascorbinsäure, ausgewählt ist.

3. Das Verfahren nach Anspruch 2, **dadurch gekennzeichnet, dass** Aktivierung unter dem Einfluss des Sauerstoff- oder Stickstoffplasmas erfolgt.

4. Das Verfahren nach den Ansprüchen 2 und 3, **dadurch gekennzeichnet, dass** Aktivierung unter Plasmadruck von 0,1-1 mbar und mit einer Plasmabetriebsdauer von 5-500 s erfolgt.

5. Das Verfahren nach Anspruch 2, **dadurch gekennzeichnet, dass** Plasma-polymerische Materialien in der Luft belassen werden, um sauerstoffhaltige funktionelle Gruppen zu bilden.

6. Das Verfahren nach Anspruch 2, **dadurch gekennzeichnet, dass** nanokristallines Titan(IV)-Oxid mit einer Korngröße bis zu 100 nm in der Form einer wässrigen kolloidalen Lösung für die Synthese verwendet wird.

7. Das Verfahren nach Anspruch 2, **dadurch gekennzeichnet, dass** das Syntheseverfahren mit der Immersionstechnik, insbesondere Tauchbeschichtungstechnik, Sprüh- oder Maltechnik, durchgeführt wird.

8. Das Verfahren nach Anspruch 2, **dadurch gekennzeichnet, dass** das Verfahren bei Raumtemperatur erfolgt.

9. Das Verfahren nach Anspruch 2, **dadurch gekennzeichnet, dass** die Oberflächenmodifizierung der Beschichtung mit organischen Verbindungen in wässriger oder alkoholischer Lösung des Modifikators mit einer Minimumkonzentration von 10⁻⁴ mol/dm³ erfolgt, gefolgt von Trocknen.

## Revendications

1. Revêtement photocatalytique d'oxyde de titane (IV) sur une surface de polymère, **caractérisé en ce que** :
a) il est appliqué sur un substrat qui est le matériau polymère organique utilisé dans un emballage médical, alimentaire et pharmaceutique et choisi dans le groupe du polyuréthanne (PU), du polycarbonate (PC), du polychlorure de vinyle (PVC), du polytétrafluoroéthylène (PTFE),
b) le revêtement est constitué d'oxyde de titane (IV) nanocristallin modifié en surface avec des composés organiques,
dans lequel l'oxyde de titane (IV) nanocristallin est modifié en surface avec un composé organique du groupe constitué par : l'acide phtalique, l'acide 4-sulfophtalique, l'acide 4-amino-2-hydroxybenzoïque, l'acide 3-hydroxy-2-naphtylique, l'acide salicylique, l'acide 6-hydroxysalicylique, l'acide 5-hydroxysalicylique, l'acide 5-sulfosalicylique, l'acide 3,5-dinitrosalicylique, le sel disodique 1,4-dihydroxy-1,3-benzènedisulfonique, l'acide gallique, le pyrogallol, le 2,3-naphtalènediol, le 4-méthylcatéchol, le 3,5-di-tert-butylcatéchol, le p-nitrocatéchol, la 3,4-dihydroxy-L-phénylalanine (DOPA), le catéchol, le rutoside et l'acide ascorbique.

2. Procédé de préparation de revêtement photocatalytique d'oxyde de titane (IV) sur la surface d'un matériau polymère utilisé dans un emballage médical, alimentaire et pharmaceutique choisi dans le groupe : du polyuréthanne (PU), du polycarbonate (PC), du polychlorure de vinyle (PVC), du polytétrafluoroéthylène (PTFE), **caractérisé en ce qu'**il comprend trois étapes :
a) activation de surface d'un matériau polymère avec une technologie au plasma à basse température,
b) synthèse d'un revêtement nanocristallin d'oxyde de titane (IV) à l'aide d'une suspension d'oxyde de titane (IV) nanocristallin,
c) modification de la surface de revêtement à l'aide de composés organiques au moyen de l'application du modificateur en solution,
dans lequel le composé organique utilisé pour la modification de surface est choisi dans le groupe constitué par l'acide phtalique, l'acide 4-sulfophtalique, l'acide 4-amino-2-hydroxybenzoïque, l'acide 3-hydroxy-2-naphtylique, l'acide salicylique, l'acide 6-hydroxysalicylique, l'acide 5-hydroxysalicylique, l'acide 5-sulfosalicylique, l'acide 3,5-dinitrosalicylique, le sel disodique 1,4-dihydroxy-1,3-benzènedisulfonique, l'acide gallique, le pyrogallol, le 2,3-naphtalènediol, le 4-méthylcatéchol, le 3,5-di-tert-butylcatéchol, le p-nitrocatéchol, la 3,4-dihydroxy-L-phénylalanine (DOPA), le catéchol, le rutoside et l'acide ascorbique.

3. Procédé selon la revendication 2, **caractérisé en ce que** l'activation se déroule sous l'influence du plasma d'oxygène ou d'azote.

4. Procédé selon les revendications 2 et 3, **caractérisé en ce que** l'activation se déroule sous une pression de plasma de 0,1 à 1 mbar et en un temps de fonctionnement du plasma de 5 à 500 s.

5. Procédé selon la revendication 2, **caractérisé en ce que** les matériaux polymères de plasma sont laissés à l'air pour former des groupes fonctionnels contenant de l'oxygène.

6. Procédé selon la revendication 2, **caractérisé en ce que** de l'oxyde de titane (IV) nanocristallin ayant une dimension de grain allant jusqu'à 100 nm sous la forme d'une solution colloïdale aqueuse est utilisé pour la synthèse.

7. Procédé selon la revendication 2, **caractérisé en ce que** le procédé de synthèse est mis en oeuvre par les techniques d'immersion, en particulier par une technique de revêtement par trempage, des techniques de pulvérisation ou de peinture.

8. Procédé selon la revendication 2, **caractérisé en ce que** le procédé se déroule à température ambiante.

9. Procédé selon la revendication 2, **caractérisé en ce que** la modification de surface du revêtement avec des composés organiques se déroule dans une solution aqueuse ou alcoolique de modificateur avec une concentration minimale de 10⁻⁴ mole/dm³ suivie d'un séchage.
